# EUROPÄISCHE PATENTANMELDUNG

(11) **EP 4 624 037 A1**
(43) Veröffentlichungstag der Anmeldung: **01.10.2025**
(21) Anmeldenummer: 24167624.6
(22) Anmeldetag: 28.03.2024
(51) Int. Cl.: B01J 8/06, F28D 7/16

(54) **STÜTZPLATTENPAAR FÜR ROHRE IN EINEM REAKTORBEHÄLTER**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Oelmann, Tobias, 60439 Frankfurt (DE); Strozyk, Michael, 60439 Frankfurt (DE); Personne, David, 60439 Frankfurt (DE); Tercan, Abdullah Enes, 60439 Frankfurt (DE); Gronemann, Veronika, 60439 Frankfurt (DE)
(74) Vertreter: Air Liquide

(57) **Zusammenfassung**

Vorrichtung (1.1;1.2) umfassend einen Reaktorbehälter (2), ein Rohrbündel (3) mit mehreren Rohren (4), eine erste Stützplatte (5) und eine zweite Stützplatte (6),
wobei das Rohrbündel (3) im Reaktorbehälter (2) angeordnet ist, wobei das Rohrbündel (3) mehrere erste Rohrgruppen (7) und mehrere zweite Rohrgruppen (8) umfasst,
wobei die erste Stützplatte (5) und die zweite Stützplatte (6) quer zu einer Längsachse (9) des Reaktorbehälters (2) im Reaktorbehälter (2) angeordnet sind, wobei die erste Stützplatte (5) zur zweiten Stützplatte (6) entlang der Längsachse (9) des Reaktorbehälters (2) versetzt ist, wobei jedes der Rohre (4) der ersten Rohrgruppen (7) durch eine jeweilige Rohröffnung (10.1) der ersten Stützplatte (5) geführt ist, und wobei die erste Stützplatte (5) mehrere Aussparungen (11.1) zum Fluidaustausch aufweist, wobei jede der zweiten Rohrgruppen (8) durch eine jeweilige der Aussparungen (11.1) in der ersten Stützplatte (5) geführt ist, wobei jedes der Rohre (4) der zweiten Rohrgruppen (8) durch eine jeweilige Rohröffnung (10.2) der zweiten Stützplatte (6) geführt ist, und wobei die zweite Stützplatte (6) mehrere Aussparungen (11.2) zum Fluidaustausch aufweist, wobei jede der ersten Rohrgruppen (7) durch eine jeweilige der Aussparungen (11.2) in der zweiten Stützplatte (6) geführt ist, wobei die erste Stützplatte (5) die Rohre (4) der ersten Rohrgruppen (7) in den Rohröffnungen (10.1) der ersten Stützplatte (5) quer zur Längsrichtung der Rohre (4) stützt und die zweite Stützplatte (6) die Rohre (4) der zweiten Rohrgruppen (8) in den Rohröffnungen (10.2) der zweiten Stützplatte (6) quer zur Längsrichtung der Rohre (4) stützt.

## Beschreibung

Die Erfindung betrifft eine Anordnung zur Methanolsynthese sowie eine Vorrichtung, welche Teil einer solchen Anordnung sein kann. Weiterhin betrifft die Erfindung eine Verwendung der Vorrichtung und ein Verfahren zur Methanolsynthese mit der Vorrichtung.

Bekannt sind Verfahren zur industriellen Herstellung von Methanol durch heterogen-katalytische Umsetzung von Synthesegas in geeigneten Synthesereaktoren. Synthesegase können unterschiedliche Gasgemische sein, die unter anderem Wasserstoff und Kohlenoxide enthalten. Der Reaktor ist meist als ein stehender Festrohrwärmetauscher ausgeführt.

Bekannt sind auch zweistufige Verfahren zur Herstellung von Methanol. Dabei wird Synthesegas einem wassergekühlten Reaktor und anschließend einem gasgekühlten Reaktor zugeführt. Mit einem kupferbasierten Festbett-Katalysator wird das Synthesegas zu Methanol umgesetzt.

Bei einem wassergekühlten Reaktor befindet sich der Katalysator innerhalb der Rohre, umgeben von Wasser oder Wasserdampf auf der Mantelseite. Die Rohre werden über Rohrplatten und Stützbleche im Reaktor mechanisch fixiert.

Die Kühlung im wassergekühlten Reaktor erfolgt über eine Wärmeabgabe ins Wasser, wobei Dampf entstehen kann. Das Dampf-Wasser-Gemisch steigt an den Rohren auf. Die Stützbleche müssen die Fixierung der Rohre sicherstellen. Bekannt sind Stützbleche, die versetzt im Reaktorbehälter eingesetzt sind, so dass das Kühlmittel mäanderförmig im Reaktorbehälter um die Stützbleche strömt.

Abhängig von den gewählten Geometrien der wärmeübertragenden Komponenten und der mechanischen Elemente der Kühlmittelseite wird ein Druckverlust auf der Kühlmittelseite im Wasserdampf erzeugt. Vor allem lange Reaktorrohre führen zu einem höheren Druckverlust.

Häufig sind die äußeren Abmessungen des Reaktors beim Transport beschränkt, so dass dies zu langen, schlanken Reaktoren führt. Daraus folgt jedoch ein erhöhter Druckverlust auf der Kühlmittelseite. Durch die lange Reaktorform werden weitere Stützplatten notwendig, um ein Durchhängen der Rohre bei Transport, sowie Durchbiegen, Knicken und Schwingen im Betrieb zu vermeiden.

Aufgabe der vorliegenden Erfindung ist es, ausgehend vom beschriebenen Stand der Technik die Hinderniswirkung der Stützplatte zu reduzieren und gleichzeitig ein Knicken, Durchbiegen oder Schwingen der Rohre zu verhindern.

Diese Aufgabe wird gelöst mit den Gegenständen der unabhängigen Ansprüche. Weitere vorteilhafte Ausgestaltungen sind in den abhängigen Ansprüchen angegeben. Die in den Ansprüchen und in der Beschreibung dargestellten Merkmale sind in beliebiger, technologisch sinnvoller Weise miteinander kombinierbar.

Erfindungsgemäß wird eine Vorrichtung vorgestellt, welche einen Reaktorbehälter, ein Rohrbündel mit mehreren Rohren, eine erste Stützplatte und eine zweite Stützplatte umfasst. Das Rohrbündel ist im Reaktorbehälter angeordnet. Das Rohrbündel umfasst mehrere erste Rohrgruppen und mehrere zweite Rohrgruppen. Die erste Stützplatte und die zweite Stützplatte sind quer zu einer Längsachse des Reaktorbehälters im Reaktorbehälter angeordnet. Die erste Stützplatte ist zur zweiten Stützplatte entlang der Längsachse des Reaktorbehälters versetzt.

Jedes der Rohre der ersten Rohrgruppen ist durch eine jeweilige Rohröffnung der ersten Stützplatte geführt, und die erste Stützplatte weist mehrere Aussparungen zum Fluidaustausch auf. Jede der zweiten Rohrgruppen ist durch eine jeweilige der Aussparungen in der ersten Stützplatte geführt.

Jedes der Rohre der zweiten Rohrgruppen ist durch eine jeweilige Rohröffnung der zweiten Stützplatte geführt, und die zweite Stützplatte weist mehrere Aussparungen zum Fluidaustausch auf. Jede der ersten Rohrgruppen ist durch eine jeweilige der Aussparungen in der zweiten Stützplatte geführt.

Die erste Stützplatte stützt die Rohre der ersten Rohrgruppen in den Rohröffnungen der ersten Stützplatte quer zur Längsrichtung der Rohre und die zweite Stützplatte stützt die Rohre der zweiten Rohrgruppen in den Rohröffnungen der zweiten Stützplatte quer zur Längsrichtung der Rohre.

Die Vorrichtung ist vorzugsweise als ein Reaktor ausgebildet. Die Vorrichtung kann zur Durchführung einer chemischen Reaktion eingerichtet sein. Bei der chemischen Reaktion kann es sich um eine exotherme oder endotherme Reaktion handeln. Besonders geeignet ist die Vorrichtung für die Methanolsynthese. Allerdings können die hierin beschriebenen Vorteile auch bei zahlreichen anderen chemischen Reaktionen erzielt werden. Die Vorteile können sogar erzielt werden, wenn die Vorrichtung nicht als Reaktor genutzt wird und in der Vorrichtung keine chemische Reaktion abläuft. Die Vorrichtung kann allgemein als Wärmetauscher ausgebildet sein.

Die Vorrichtung umfasst einen Reaktorbehälter und ein darin angeordnetes Rohrbündel mit mehreren Rohren. Durch die Rohre kann ein erstes Medium strömen. Außerhalb der Rohre kann sich ein zweites Medium in dem Reaktorbehälter befinden. Das zweite Medium kann durch Zwischenräume zwischen den Rohren strömen. Zwischen dem ersten Medium und dem zweiten Medium kann ein Wärmetausch stattfinden. Das erste Medium und das zweite Medium können in einander entgegengesetzte Richtungen strömen. In dem Fall wird die Vorrichtung im Gegenstrombetrieb betrieben. Das erste Medium und das zweite Medium können aber auch in die gleiche Richtung strömen. Im Falle der Methanolsynthese kann das erste Medium beispielsweise die Reaktionsedukte enthalten und das zweite Medium ein Kühlmedium sein, oder umgekehrt.

Vorzugsweise ist der Reaktorbehälter ein länglicher Hohlkörper. Der Reaktorbehälter kann ein zylindrischer Metallbehälter sein, der dazu geeignet ist, das Rohrbündel zu umfassen. Der Reaktorbehälter kann Anschlüsse und Verbindungen aufweisen, so dass das Rohrbündel fluidtechnisch angeschlossen werden kann. Weiterhin kann der Reaktorbehälter Anschlüsse und Verbindungen aufweisen, so dass in einen Mantelraum außerhalb und zwischen den Rohren ein Kühlmedium oder sonstiges Medium in den Reaktorbehälter eingeleitet und wieder abgeleitet werden kann. Der Mantelraum kann fluidtechnisch angeschlossen werden.

Das Rohrbündel umfasst mehrere Rohre. Das Rohrbündel umfasst vorzugsweise mindestens 18 Rohre. Bevorzugt umfasst das Rohrbündel mindestens 100 Rohre. Besonders bevorzugt umfasst das Rohrbündel sogar mindestens 1000 Rohre.

Das Rohrbündel umfasst mehrere erste Rohrgruppen und mehrere zweite Rohrgruppen.

Insbesondere können die ersten Rohrgruppen jeweils zwischen 9 und 36 der Rohre umfassen und/oder die zweiten Rohrgruppen können jeweils zwischen 9 und 36 der Rohre umfassen.

Die Rohre können längliche zylindrische Hohlkörper mit einer gleichmäßigen Wanddicke sein. Die Wanddicke der Rohre beträgt vorzugsweise höchstens 5 mm, bevorzugt höchstens 3 mm und besonders bevorzugt höchstens 0,1 mm. Insbesondere kann die Wanddicke der Rohre einer für die Anwendung genormten Wanddicke entsprechen. Die Rohre können insbesondere aus einem metallischen Material gefertigt sein. Bevorzugt ist das Material wärmeleitend.

Vorzugsweise beinhalten einige der Rohre oder alle Rohre einen Katalysator. Im Falle der Methanolsynthese können so beispielsweise die Reaktionsedukte durch die Rohre geleitet werden und in den Rohren mit dem Katalysator zur Reaktion gebracht werden, so dass Methanol entsteht.

Die Reaktionsedukte können insbesondere in Form eines Synthesegases bereitgestellt werden. Vorzugsweise sind Wasserstoff, Kohlenmonoxid und Kohlenstoffdioxid Reaktionsedukte. Bevorzugt ist ein Gemisch aus Wasserstoff und Kohlenmonoxid. Besonders bevorzugt ist ein Gemisch aus Wasserstoff und Kohlenstoffdioxid. Weiterhin kann das Synthesegas Inertgase enthalten. Insbesondere kann das Synthesegas Methan als Inertgas enthalten. Bevorzugt enthält das Synthesegas Stickstoff als Inertgas. Das Synthesegas kann auf einer Einlassseite in die Rohre geleitet werden und mit dem Katalysator im Rohr reagieren. Im Falle einer exothermen Reaktion kann bei der Reaktion entstehende Wärme über den Rohrmantel abgeleitet werden. Ein Kühlmittel kann die Wärme, bevorzugt konvektiv, von der Mantelseite abführen. Das im Rohr entstandene Produkt der Reaktion kann zusammen mit dem verbleibenden Synthesegas auf der Auslassseite abgeführt werden.

Alternativ kann ein Synthesegas mit den Reaktionsedukten auch außerhalb der Rohre durch den Reaktorbehälter geleitet werden. Insbesondere in dem Fall kann eine Katalysatorschüttung auf der Mantelseite im Mantelraum vorgesehen sein. Das Synthesegas kann dann durch die Katalysatorschüttung geführt werden. Ein Kühlmittel kann auf einer Einlassseite in die Rohre geleitet werden. Die durch die Reaktion erzeugte Wärme kann über die Mantelseite der Rohre an das Kühlmittel in den Rohren übertragen werden. Das erwärmte Kühlmittel kann auf einer Auslassseite der Rohre abgeführt werden.

Weiterhin umfasst die Vorrichtung eine erste Stützplatte und eine zweite Stützplatte. Die erste Stützplatte und/oder die zweite Stützplatte können als ein Blech ausgebildet sein. Vorzugsweise haben die erste Stützplatte und/oder die zweite Stützplatte eine Dicke von mindestens 7 mm. Bevorzugt ist die Dicke der ersten Stützplatte und/oder der zweiten Stützplatte höchstens halb so groß wie ein Durchmesser der Rohre. Die optimale Dicke der Stützplatte kann darüber hinaus über Berechnungen ermittelt werden. Haben nicht alle Rohre den gleichen Durchmesser, ist die Dicke der ersten Stützplatte und/oder der zweiten Stützplatte vorzugsweise höchstens halb so groß wie der größte Durchmesser der Rohre des Rohrbündels.

Die erste Stützplatte und die zweite Stützplatte sind quer zu einer Längsachse des Reaktorbehälters im Reaktorbehälter angeordnet. Insbesondere können die erste Stützplatte und die zweite Stützplatte im Reaktorbehälter befestigt sein.

Die Längsachse des Reaktorbehälters ist bevorzugt entlang der länglichen Richtung des Hohlkörpers ausgerichtet. Insbesondere ist die erste Stützplatte und/oder die zweite Stützplatte senkrecht zur Längsachse des Reaktorbehälters im Reaktorbehälter angeordnet. Vorzugsweise ist der Reaktorbehälter und/oder das Rohrbündel vertikal ausgerichtet. Die "und"-Ausführungen sind bevorzugt.

Die erste Stützplatte ist zur zweiten Stützplatte entlang der Längsachse des Reaktorbehälters versetzt.

Vorzugsweise weist die erste Stützplatte einen Abstand entlang der Längsachse des Reaktorbehälters von mindestens 100 mm zur zweiten Stützplatte auf. Bevorzugt weist die erste Stützplatte einen Abstand entlang der Längsachse des Reaktorbehälters von mindestens 350 mm zur zweiten Stützplatte auf. Besonders bevorzugt weist die erste Stützplatte einen Abstand entlang der Längsachse des Reaktorbehälters von mindestens 700 mm zur zweiten Stützplatte auf.

Vorzugsweise weist die erste Stützplatte einen Abstand entlang der Längsachse des Reaktorbehälters von höchstens 2000 mm zur zweiten Stützplatte auf.

Jedes der Rohre der ersten Rohrgruppen ist durch eine jeweilige Rohröffnung der ersten Stützplatte geführt, und die erste Stützplatte weist mehrere Aussparungen zum Fluidaustausch auf. Jede der zweiten Rohrgruppen ist durch eine jeweilige der Aussparungen in der ersten Stützplatte geführt. Jedes der Rohre der zweiten Rohrgruppen ist durch eine jeweilige Rohröffnung der zweiten Stützplatte geführt, und die zweite Stützplatte weist mehrere Aussparungen zum Fluidaustausch auf. Jede der ersten Rohrgruppen ist durch eine jeweilige der Aussparungen in der zweiten Stützplatte geführt.

Insbesondere sind die Rohröffnungen der ersten Stützplatte den Rohren der ersten Rohrgruppen zugeordnet und die Rohröffnungen der zweiten Stützplatte sind den Rohren der zweiten Rohrgruppen zugeordnet.

Die erste Stützplatte und die zweite Stützplatte können den Fluidaustausch im Mantelraum des Reaktorbehälters beeinträchtigen. Um diesen Effekt so gering wie möglich zu halten, weisen die erste Stützplatte und die zweite Stützplatte jeweils mehrere Aussparungen auf. Der Fluidaustausch findet insbesondere zwischen Bereichen des Reaktorbehälters statt, zwischen denen die erste Stützplatte und die zweite Stützplatte angeordnet sind.

Jedes der Rohre des Rohrbündels gehört entweder zu genau einer der ersten Rohrgruppen, zu genau einer der zweiten Rohrgruppen oder zu keiner der Rohrgruppen. Auch wenn es Rohre gibt, die zu keiner der Rohrgruppen gehören, gibt es jedenfalls kein Rohr, welches zu mehreren der Rohrgruppen zugleich gehört. Die Rohrgruppen sind allein durch die hierin beschriebenen Eigenschaften definiert. Es ist nicht erforderlich, dass die Zuordnung der Rohre zu den Rohrgruppen darüber hinaus an strukturellen Merkmalen der Vorrichtung zu erkennen ist.

Die Rohre aller ersten Rohrgruppen sind durch eine jeweilige Rohröffnung der ersten Stützplatte geführt. Jedes Rohr hat dabei eine eigene Rohröffnung. Dadurch werden die Rohre der ersten Rohrgruppe mit der ersten Stützplatte gestützt. Die Rohre der ersten Rohrgruppen sind zudem durch eine der Aussparungen der zweiten Stützplatte geführt. Dabei ist jede der ersten Rohrgruppen durch eine jeweilige der Aussparungen in der zweiten Stützplatte geführt. Jede erste Rohrgruppe hat dabei eine eigene Aussparung. Alle Rohre einer ersten Rohrgruppe sind gemeinsam durch diese Aussparung geführt. Die ersten Rohrgruppen umfassen also jeweils all die Rohre, die gemeinsam durch eine der Aussparungen der zweiten Stützplatte geführt werden. Die Rohre der ersten Rohrgruppen werden im Allgemeinen nicht durch die zweite Stützplatte gestützt. Stattdessen passieren diese Rohre die zweite Stützplatte lediglich, indem die Rohre durch die entsprechende Aussparung geführt sind. Die Aussparungen in der zweiten Stützplatte dienen dem Fluidaustausch.

Die Rohre aller zweiten Rohrgruppen sind durch eine jeweilige Rohröffnung der zweiten Stützplatte geführt. Jedes Rohr hat dabei eine eigene Rohröffnung. Dadurch werden die Rohre der zweiten Rohrgruppe mit der zweiten Stützplatte gestützt. Die Rohre der zweiten Rohrgruppen sind zudem durch eine der Aussparungen der ersten Stützplatte geführt. Dabei ist jede der zweiten Rohrgruppen durch eine jeweilige der Aussparungen in der ersten Stützplatte geführt. Jede zweite Rohrgruppe hat dabei eine eigene Aussparung. Alle Rohre einer zweiten Rohrgruppe sind gemeinsam durch diese Aussparung geführt. Die zweiten Rohrgruppen umfassen also jeweils all die Rohre, die gemeinsam durch eine der Aussparungen der ersten Stützplatte geführt werden. Die Rohre der zweiten Rohrgruppen werden im Allgemeinen nicht durch die erste Stützplatte gestützt. Stattdessen passieren diese Rohre die erste Stützplatte lediglich, indem die Rohre durch die entsprechende Aussparung geführt sind. Die Aussparungen in der ersten Stützplatte dienen dem Fluidaustausch. Falls es ein Rohr gibt, welches weder zur ersten Rohrgruppe noch zur zweiten Rohrgruppe gehört, kann dieses Rohr beispielsweise sowohl in der ersten Stützplatte als auch in der zweiten Stützplatte durch eine jeweilige Rohröffnung geführt sein. Ein solches Rohr ist dann sowohl durch die erste Stützplatte als auch durch die zweite Stützplatte gestützt.

Jedes der Rohre ist also entweder zumindest durch die erste Stützplatte oder zumindest durch die zweite Stützplatte gestützt. Das Zusammenspiel von erster Stützplatte und zweiter Stützplatte bewirkt also die gewünschte Stützung aller Rohre. Die erste Stützplatte und die zweite Stützplatte können insoweit als eine Stützeinheit aufgefasst werden. Die hierin beschriebenen Vorteile werden bereits erzielt, wenn die Vorrichtung eine solche Stützeinheit aufweist. Es ist aber auch möglich und sogar bevorzugt, dass die Vorrichtung mehrere Stützeinheiten aufweist, welche jeweils eine erste Stützplatte und eine zweite Stützplatte aufweisen. Die Stützeinheiten sind vorzugsweise jeweils wie hierin beschrieben ausgebildet.

Die Aussparungen in der ersten Stützplatte und in der zweiten Stützplatte liegen vorzugsweise nicht deckungsgleich übereinander. Die Aussparungen können aber dennoch teilweise überlappen. In einem Zwischenraum zwischen der ersten Stützplatte und der zweiten Stützplatte kann das Fluid von den Aussparungen der ersten Stützplatte zu den Aussparungen der zweiten Stützplatte oder umgekehrt strömen. Dies kann mäandrierende Strömungen des Fluids erzeugen, die teilweise quer zur Längsachse verlaufen.

Ein freier Strömungsquerschnitt im Reaktorbehälter kann durch die Aussparungen in der ersten Stützplatte und in der zweiten Stützplatte bestimmt werden. Der freie Strömungsquerschnitt ist der Querschnitt eines Rohres oder Kanals, durch den ein Medium strömt. Der freie Strömungsquerschnitt bezeichnet also eine Fläche, durch die ein Medium strömen kann. Je größer die kleinste Gesamtfläche aller Aussparungen der ersten Stützplatte oder der zweiten Stützplatte, desto größer ist der freie Strömungsquerschnitt.

Die erste Stützplatte stützt die Rohre der ersten Rohrgruppen in den Rohröffnungen der ersten Stützplatte quer zur Längsrichtung der Rohre und die zweite Stützplatte stützt die Rohre der zweiten Rohrgruppen in den Rohröffnungen der zweiten Stützplatte quer zur Längsrichtung der Rohre. Dies hat den Vorteil, dass die Rohre des Rohrbündels sich individuell thermisch ausdehnen können, aber Schwingungen durch bestimmte Strömungszustände und ein Knicken der Rohre vermieden werden können. Ein weiterer Vorteil ist, dass alle Rohre des Rohrbündels von einer ersten Stützplatte zusammen mit einer zweiten Stützplatte gestützt sind.

Vorzugsweise sind die Rohre des Rohrbündels in axialer Richtung in der jeweiligen Rohröffnung der ersten Stützplatte und der zweiten Stützplatte nicht fixiert. Die Rohre der ersten Rohrgruppe sind in dem Fall in axialer Richtung durch eine jeweilige Rohröffnung der ersten Stützplatte axial bewegbar. Die Rohre der zweiten Rohrgruppe sind in demselben Fall in axialer Richtung durch eine jeweilige Rohröffnung der zweiten Stützplatte axial bewegbar. Lokal unterschiedliche Temperaturen können lokal unterschiedliche Ausdehnungen der Rohre zur Folge haben. Dadurch, dass die Rohre axial bewegbar sind, werden einzelne Rohre nicht durch eine der Stützplatten in ihrer individuellen Ausdehnung gestört. Bewegungen quer zur Längsachse werden jedoch minimiert.

Die Form der ersten Stützplatte und der zweiten Stützplatte kann der des Reaktorbehälters angepasst sein. Vorzugsweise weist die erste Stützplatte und/oder die zweite Stützplatte eine rechteckige Form mit abgerundeten Ecken auf. Besonders bevorzugt weist die erste Stützplatte und/oder die zweite Stützplatte eine elliptische Form, insbesondere eine kreisförmige Form auf. Die "und"-Ausführungen sind bevorzugt.

Die Vorrichtung hat den Vorteil, dass die Hinderniswirkung der ersten Stützplatte und der zweiten Stützplatte minimiert wird und gleichzeitig ein Knicken, Durchbiegen oder Schwingen der Rohre während des Transports oder während des Betriebs verhindert wird.

In einer bevorzugten Ausführungsform sind die Aussparungen in der ersten Stützplatte und/oder die Aussparungen in der zweiten Stützplatte rautenförmig.

Die Aussparungen der ersten Stützplatte können bei 9 Rohren pro zweiter Rohrgruppe 3 mal 3 Rohre rautenförmig umfassen. Die Aussparungen der zweiten Stützplatte können bei 9 Rohren pro erster Rohrgruppe 3 mal 3 Rohre rautenförmig umfassen.

Die Aussparungen der ersten Stützplatte können bei 16 Rohren pro zweiter Rohrgruppe 4 mal 4 Rohre rautenförmig umfassen. Die Aussparungen der zweiten Stützplatte können bei 16 Rohren pro erster Rohrgruppe 4 mal 4 Rohre rautenförmig umfassen.

Die Aussparungen der ersten Stützplatte können bei 25 Rohren pro zweiter Rohrgruppe 5 mal 5 Rohre rautenförmig umfassen. Die Aussparungen der zweiten Stützplatte können bei 25 Rohren pro erster Rohrgruppe 5 mal 5 Rohre rautenförmig umfassen.

Die Aussparungen der ersten Stützplatte können bei 36 Rohren pro zweiter Rohrgruppe 6 mal 6 Rohre rautenförmig umfassen. Die Aussparungen der zweiten Stützplatte können bei 36 Rohren pro erster Rohrgruppe 6 mal 6 Rohre rautenförmig umfassen.

Der Vorteil dieser Ausführungsform liegt darin, dass der freie Strömungsquerschnitt durch die rautenförmigen Ausschnitte größer ist und die Strömung durch die erste Strömungsplatte und durch die zweite Strömungsplatte möglichst wenig gestört wird.

In einer weiteren bevorzugten Ausführungsform sind jeweils mehrere der ersten Rohrgruppen in einer ersten Reihe nebeneinander angeordnet, wobei jeweils mehrere der zweiten Rohrgruppen in einer zweiten benachbarten Reihe nebeneinander angeordnet sind, wobei erste und zweite Reihen alternierend angeordnet sind.

Nachfolgend wird eine Betrachtungsweise, in der nur die Aussparungen der ersten und der zweiten Stützplatte berücksichtigt werden, und erste Stützplatte und die zweite Stützplatte in einer Ebene überlagert werden, überlagerte Betrachtungsweise genannt.

In einer überlagerten Betrachtungsweise können dementsprechend jeweils mehrere der Aussparungen der zweiten Stützplatte in einer ersten Reihe nebeneinander angeordnet sein, wobei jeweils mehrere der Aussparungen der ersten Stützplatte in einer zweiten benachbarten Reihe nebeneinander angeordnet sind, wobei erste und zweite Reihen alternierend angeordnet sind. Dies kann auch als ein Muster bezeichnet werden.

Diese Ausführungsform hat den Vorteil, dass der Druckverlust durch Strömungswiderstände an der ersten Stützplatte und an der zweiten Stützplatte reduziert wird. Dies liegt daran, dass das Fluid zwischen der ersten Stützplatte und der zweiten Stützplatte in dieser Ausführungsform weniger abgelenkt wird.

In einer weiteren bevorzugten Ausführungsform weisen die Aussparungen in der ersten Stützplatte jeweils eine sechseckige Form auf und/oder die Aussparung in der zweiten Stützplatte weisen jeweils eine sechseckige Form auf.

Insbesondere kann die jeweilige sechseckige Form länglich ausgestaltet sein, mit 2 kurzen gegenüberliegenden Seiten und 4 langen Seiten. Diese Form kann auch als eine abgestumpfte Raute beschrieben werden.

Vorzugsweise sind die ersten Rohrgruppen und zweiten Rohrgruppen in einem Schachbrettmuster alternierend angeordnet.

Insbesondere kann eine zweite Rohrgruppe von vier ersten Rohrgruppen und/oder eine erste Rohrgruppe von vier zweiten Rohrgruppen umgeben sein.

Dementsprechend können bei einer überlagerten Betrachtungsweise jeweils mehrere der Aussparungen der zweiten Stützplatte zusammen mit mehreren Aussparungen der ersten Stützplatte in einem Schachbrettmuster alternierend angeordnet sein.

Der Vorteil dieser Ausführungsform ist, dass ein geringerer Druckverlust durch die erste und die zweite Stützplatte erreicht wird, da in dieser Ausführungsform ein kürzerer Fließweg der Strömung sich einstellt. Darüber hinaus bildet sich eine symmetrische Strömung zwischen den Stützplatten aus, was sich wiederum in einer gleichmäßigen Kraft auf die Stützplatten äußert.

In einer weiteren Ausführungsform kann jeweils eine erste Rohrgruppe von sechs zweiten Rohrgruppen umgeben sein. Dementsprechend können bei einer überlagerten Betrachtungsweise, sechs Aussparung der ersten Stützplatte zur Durchführung der sechs zweiten Rohrgruppen um die eine Aussparung der zweiten Stützplatte zur Durchführung der einen ersten Rohrgruppe herum angeordnet sein. Denkbar ist auch, dass jeweils eine zweite Rohrgruppe von sechs ersten Rohrgruppen umgeben sein kann. Dementsprechend können bei einer überlagerten Betrachtungsweise sechs Aussparung der zweiten Stützplatte zur Durchführung der sechs ersten Rohrgruppen um die eine Aussparung der ersten Stützplatte zur Durchführung der einen zweiten Rohrgruppe herum angeordnet sein.

Der Vorteil dieser Ausführungsform ist, dass ein geringerer Druckverlust durch den Fluidaustausch durch die erste und die zweite Stützplatte erreicht wird, da in dieser Ausführungsform ein kürzerer Fließweg der Strömung sich einstellt. Darüber hinaus bildet sich eine symmetrische Strömung zwischen den Stützplatten aus, was sich wiederum in einer gleichmäßigen Kraft auf die Stützplatten äußert.

Weiterhin hat diese Ausführungsform den Vorteil, dass die Belastung der Strömung auf die Kanten der Aussparungen reduziert werden kann, wodurch der Druckverlust und die mechanische Belastung auf die Stützplatten weiter reduziert werden kann.

In einer weiteren bevorzugten Ausführungsform weist die erste Stützplatte zusätzlich zu den Aussparungen Ausschnitte zum Fluidaustausch auf und/oder die zweite Stützplatte weist zusätzlich zu den Aussparungen Ausschnitte zum Fluidaustausch auf.

Diese Ausführungsform hat den Vorteil, dass der Druckverlust durch Strömungswiderstände reduziert wird.

In einer weiteren bevorzugten Ausführungsform bildet die erste Stützplatte für zumindest einige der Ausschnitte der ersten Stützplatte jeweils zwischen dem Ausschnitt und den dazu nächstliegenden der Rohröffnungen der ersten Stützplatte einen jeweiligen Steg aus, und wobei die Stege jeweils eine minimale Breite haben, welche bei zumindest einigen der Stege gleich groß ist und/oder die zweite Stützplatte bildet für zumindest einige der Ausschnitte der zweiten Stützplatte jeweils zwischen dem Ausschnitt und den dazu nächstliegenden der Rohröffnungen der zweiten Stützplatte einen jeweiligen Steg aus, und wobei die Stege jeweils eine minimale Breite haben, welche bei zumindest einigen der Stege gleich groß ist.

Die erste Stützplatte und die zweite Stützplatte sollen einerseits so stabil ausgebildet sein, dass die Rohre ausreichend gestützt werden. Dies kann dadurch erreicht werden, dass die Stege eine ausreichende Breite haben. Andererseits soll die Stützplatte den Fluidaustausch möglichst wenig beeinträchtigen. Dies kann dadurch erreicht werden, dass die Stege möglichst klein gehalten sind. Durch Abwägung der beiden genannten Bedingungen kann bestimmt werden, wie breit ein Steg an seiner engsten Stelle optimalerweise sein sollte, wie groß also die minimale Breite eines Steges optimalerweise sein sollte. In der vorliegenden Ausführungsform haben zumindest einige der der Stege, vorzugsweise sogar alle der Stege die gleiche minimale Breite. Damit kann für alle diese Stege ein bestmöglicher Kompromiss zwischen Stabilität und Fluidaustausch erzielt werden.

In einer weiteren bevorzugten Ausführungsform liegen zumindest einige der Ausschnitte der ersten Stützplatte jeweils zentral zwischen drei paarweise zueinander benachbarten der Rohröffnungen der ersten Stützplatte und/oder zumindest einige der Ausschnitte der zweiten Stützplatte liegen jeweils zentral zwischen drei paarweise zueinander benachbarten der Rohröffnungen der zweiten Rohrgruppen der zweiten Stützplatte.

Die Ausschnitte der Stützplatten können beispielsweise jeweils zentral zwischen drei benachbarten Rohröffnungen liegen, wobei die Mittelpunkte dieser drei Rohröffnungen bevorzugt ein gleichseitiges Dreieck bilden.

In einer weiteren bevorzugten Ausführungsform sind zumindest einige der Ausschnitte der ersten Stützplatte Durchgangsbohrungen und/oder zumindest einige der Ausschnitte der zweiten Stützplatte sind Durchgangsbohrungen.

Der Vorteil von Durchgangsbohrungen ist, dass die erste und die zweite Stützplatte besonders einfach hergestellt werden können und eine sehr hohe Festigkeit und Steifigkeit aufweisen.

In einer weiteren bevorzugten Ausführungsform sind zumindest einige der Ausschnitte der ersten Stützplatte sternförmig und/oder zumindest einige der Ausschnitte der zweiten Stützplatte sind sternförmig.

Ein sternförmiger Ausschnitt ist hier im mathematischen Sinne zu verstehen. Ein jeweiliger Ausschnitt ist sternförmig, wenn es einen Punkt, den sog. Sternmittelpunkt, gibt, von dem aus alle weiteren Punkte des Ausschnitts sichtbar sind. Das heißt, dass jede Verbindungsstrecke eines Punktes mit dem Sternmittelpunkt vollständig in dem Ausschnitt liegt. Der Sternmittelpunkt kann bildlich gesprochen alle Seiten des Ausschnitts "sehen."

Vorzugsweise weisen zumindest einige der Ausschnitte der ersten Stützplatte und zumindest einige der Ausschnitte der zweiten Stützplatte die Form von triangulären Löchern auf. Ein trianguläres Loch meint hier ein dreieckiges Loch mit abgerundeten Ecken und konkaven Seiten. Ein trianguläres Loch ist sternförmig im hierin verwendeten Sinne des Begriffs sternförmig.

Der Vorteil dieser Ausführungsform ist, dass ohne weitere Änderungen die Fläche des Ausschnitts erhöht werden kann. Bildlich gesprochen kann der verfügbare Platz zwischen den Rohröffnungen besser genutzt werden. Die Hinderniswirkung der ersten Stützplatte und der zweiten Stützplatte kann somit weiter reduziert werden.

In einer weiteren bevorzugten Ausführungsform weist die erste Stützplatte Zwischenbereiche auf, welche jeweils zwischen drei paarweise zueinander benachbarten der Rohröffnungen ausgebildet sind, und wobei zumindest einige der Ausschnitte der ersten Stützplatte auf einen jeweiligen der Zwischenbereiche beschränkt sind. Zusätzlich oder alternativ weist die zweite Stützplatte Zwischenbereiche auf, welche jeweils zwischen drei paarweise zueinander benachbarten der Rohröffnungen ausgebildet sind, und wobei zumindest einige der Ausschnitte der zweiten Stützplatte auf einen jeweiligen der Zwischenbereiche beschränkt sind.

Der Vorteil dieser Ausführungsform ist, dass bei sonst unveränderten Bedingungen die Fläche des Ausschnitts erhöht werden kann. Bildlich gesprochen kann der Zwischenbereich zwischen den Rohröffnungen besser genutzt werden. Die Hinderniswirkung der Stützplatte kann somit weiter reduziert werden.

In einer weiteren bevorzugten Ausführungsform weist die erste Stützplatte Zwischenbereiche auf, welche jeweils zwischen drei paarweise zueinander benachbarten der Rohröffnungen ausgebildet sind, und wobei sich zumindest einige der Ausschnitte der ersten Stützplatte jeweils über zwei der Zwischenbereiche erstrecken. Zusätzlich oder alternativ weist die zweite Stützplatte Zwischenbereiche auf, welche jeweils zwischen drei paarweise zueinander benachbarten der Rohröffnungen ausgebildet sind, und wobei sich zumindest einige der Ausschnitte der zweiten Stützplatte jeweils über zwei der Zwischenbereiche erstrecken.

Diese Ausgestaltung kann erhalten werden, indem zwischen zwei benachbarten Zwischenbereichen jeweils die Verbindungsstege entfernt werden. Um eine Rohröffnung können in dieser Ausgestaltung beispielsweise drei Ausschnitte gebildet sein. Pro Ausschnitt können jeweils zwei benachbarte Zwischenbereiche verbunden werden. Jeder Ausschnitt kann vier benachbarte Rohröffnungen haben. Dies kann für die erste und/oder die zweite Stützplatte angewandt werden.

Der Vorteil dieser Ausführungsform ist, dass die Hinderniswirkung der Stützplatte reduziert werden kann. Weiterhin können die Stützplatten durch diese Ausführungsform Gewicht einsparen.

Als ein weiterer Aspekt der Erfindung wird eine Anordnung zur Methanolsynthese vorgestellt. Die Anordnung umfasst die beschriebene Vorrichtung.

Die beschriebenen Vorteile und Merkmale der Vorrichtung sind auf die Anordnung anwendbar und übertragbar.

Zum Erzeugen von Methanol kann ein Synthesegas durch Umsetzen an Katalysatoren zu Methanol umgesetzt werden. Das Synthesegas kann Reaktionsedukte für die Methanolsynthese enthalten.

Neben der beschriebenen Vorrichtung umfasst die Anordnung vorzugsweise eine Quelle für Reaktionsedukte für die Methanolsynthese. Die Anordnung kann auch mehrere wie beschrieben ausgebildete Vorrichtungen aufweisen, welche beispielsweise in Reihe geschaltet sind.

Als ein weiterer Aspekt der Erfindung wird ein Verfahren vorgestellt. In dem Verfahren werden Reaktionsedukte für die Methanolsynthese durch die Rohre des Rohrbündels geleitet. Ein Kühlmedium wird außerhalb der Rohre des Rohrbündels durch den Reaktorbehälter geleitet. Vorzugsweise ist das Kühlmedium flüssiges Wasser und/oder Wasserdampf.

In einer alternativen Ausführung des Verfahrens können die Reaktionsedukte für die Methanolsynthese um die Rohre des Rohrbündels durch den Reaktorbehälter geleitet werden. Ein Kühlmedium wird dann durch die Rohre des Rohrbündels geleitet. Vorzugsweise ist das Kühlmedium gasförmig.

Die beschriebenen Vorteile und Merkmale der Vorrichtung sind auf das Verfahren anwendbar und übertragbar, und umgekehrt. Die Vorrichtung ist vorzugsweise zum Betrieb gemäß dem beschriebenen Verfahren eingerichtet. Das Verfahren wird vorzugsweise mit der Vorrichtung durchgeführt.

Als ein weiterer Aspekt der Erfindung wird eine Verwendung vorgestellt. Die beschriebene Vorrichtung wird dabei zur Methanolsynthese verwendet. Reaktionsedukte werden in der Vorrichtung zu Methanol umgesetzt.

Die beschriebenen Vorteile und Merkmale der Vorrichtung, der Anordnung und des Verfahrens sind auf die Verwendung anwendbar und übertragbar, und umgekehrt.

Vorzugsweise wird die Vorrichtung für die wassergekühlte Methanolsynthese verwendet. Alternativ ist bevorzugt, dass die Vorrichtung für die gasgekühlte Methanolsynthese verwendet wird.

In der wassergekühlten Methanolsynthese werden die Rohre mit Wasser gekühlt, während ein Synthesegas in den Rohren zu Methanol umgesetzt wird. Das Wasser führt die Wärme von den Rohren ab und verdampft dabei teilweise. Die aufsteigenden Dampfblasen können durch die Aussparungen der ersten Stützplatte und durch die Aussparungen der zweiten Stützplatte, oder umgekehrt, strömen. Insbesondere können die aufsteigenden Dampfblasen durch die Ausschnitte der ersten Stützplatte und durch die Ausschnitte der zweiten Stützplatte, oder umgekehrt, strömen.

In der gasgekühlten Methanolsynthese werden die Rohre mit einem kühleren Gas in den Rohren gekühlt. Das Synthesegas wird außerhalb der Rohre zu Methanol umgesetzt. Dabei wird die Wärme an die Rohre übertragen und durch das Gas in den Rohren abgeführt. Das Synthesegas kann durch die Aussparungen der ersten Stützplatte und durch die Aussparungen der zweiten Stützplatte, oder umgekehrt, strömen. Insbesondere kann das Synthesegas durch die Ausschnitte der ersten Stützplatte und durch die Ausschnitte der zweiten Stützplatte, oder umgekehrt, strömen.

Die Erfindung wird nachfolgend anhand der Figuren näher erläutert. Die Figuren zeigen ein besonders bevorzugtes Ausführungsbeispiel, auf das die Erfindung jedoch nicht begrenzt ist. Die Figuren und die darin dargestellten Größenverhältnisse sind nur schematisch. Es zeigen:
- Fig. 1:: eine schematische Ansicht einer erfindungsgemäßen Anordnung zur Methanolsynthese,
- Fig. 2:: eine schematische Draufsicht auf eine erste Ausgestaltung einer ersten Stützplatte, wie sie bei der Anordnung aus Fig. 1 verwendet werden kann,
- Fig. 3:: eine schematische Draufsicht auf eine erste Ausgestaltung einer zweiten Stützplatte, wie sie bei der Anordnung aus Fig. 1 verwendet werden kann,
- Fig. 4:: einen Ausschnitt einer ersten Stützplatte in einer ersten Ausgestaltung aus Fig. 2,
- Fig. 5:: einen Ausschnitt einer zweiten Stützplatte in einer ersten Ausgestaltung aus Fig. 3,
- Fig. 6:: eine schematische Draufsicht auf eine erste Stützplatte mit Aussparungen und auf eine zweite Stützplatte mit Aussparungen in einer überlagerten Betrachtungsweise in einer ersten Ausgestaltung aus Fig. 2 bis Fig. 5, wie sie bei der Anordnung aus Fig. 1 verwendet werden kann,
- Fig. 7:: eine Detailansicht der ersten Ausgestaltung der Aussparungen der ersten und zweiten Stützplatten in einer überlagerten Betrachtungsweise aus Fig. 6,
- Fig. 8:: eine Detailansicht einer zweiten Ausgestaltung der Aussparungen der ersten Stützplatte und der zweiten Stützplatte in einer überlagerten Betrachtungsweise, wie sie bei der Anordnung aus Fig. 1 verwendet werden kann,
- Fig. 9:: eine Detailansicht einer dritten Ausgestaltung der Aussparungen der ersten Stützplatte und der zweiten Stützplatte in einer überlagerten Betrachtungsweise, wie sie bei der Anordnung aus Fig. 1 verwendet werden kann,
- Fig. 10:: eine Detailansicht einer vierten Ausgestaltung der ersten und zweiten Stützplatte, wie sie bei der Anordnung aus Fig. 1 verwendet werden kann,
- Fig. 11:: eine Detailansicht einer fünften Ausgestaltung der ersten und zweiten Stützplatte, wie sie bei der Anordnung aus Fig. 1 verwendet werden kann,
- Fig. 12:: eine Detailansicht einer sechsten Ausgestaltung der ersten und zweiten Stützplatte, wie sie bei der Anordnung aus Fig. 1 verwendet werden kann.
- Fig. 13:: eine Detailansicht einer siebten Ausgestaltung der Aussparungen und der Ausschnitte der ersten Stützplatte, wie sie bei der Anordnung aus Fig. 1 verwendet werden kann,
- Fig. 14:: eine Detailansicht einer siebten Ausgestaltung der Aussparungen und der Ausschnitte der ersten Stützplatte aus Fig. 13.

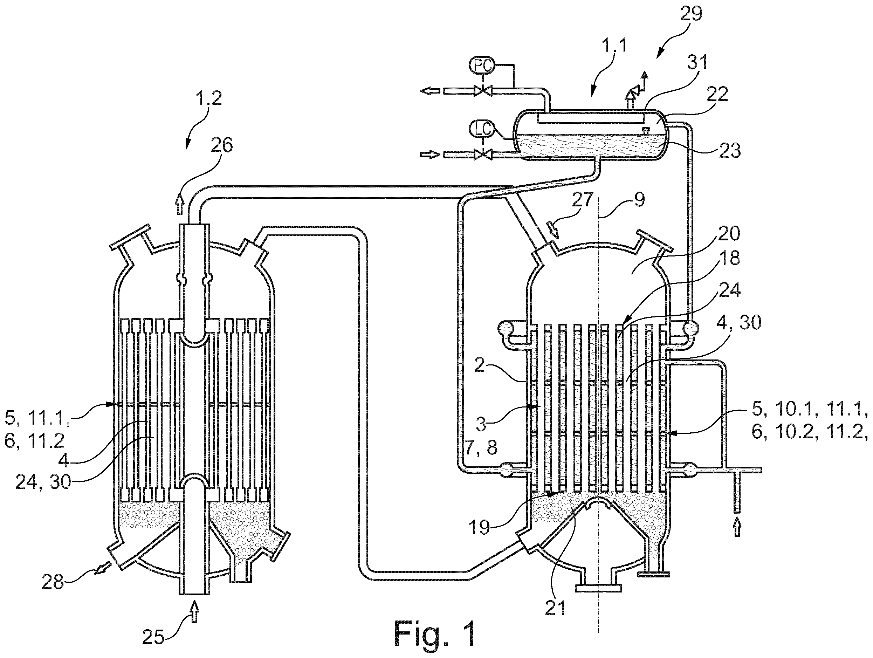

Fig. 1 zeigt eine schematische Ansicht einer Anordnung 29 zur Methanolsynthese. Dabei wird Synthesegas durch zwei in Serie geschaltete Vorrichtungen 1.1;1.2 geleitet. Die Vorrichtungen 1.1;1.2 werden zur Methanolsynthese verwendet.

Vorgeheiztes Synthesegas wird durch einen Gaseinlass 27 der ersten Vorrichtung 1.1 zugeführt.

Die erste Vorrichtung 1.1 umfasst einen Reaktorbehälter 2, ein Rohrbündel 3 mit mehreren Rohren 4, eine erste Stützplatte 5 und eine zweite Stützplatte 6. Das Rohrbündel 3 ist im Reaktorbehälter 2 angeordnet. Das Rohrbündel 3 umfasst mehrere erste Rohrgruppen 7 und mehrere zweite Rohrgruppen 8. Die erste Stützplatte 5 und die zweite Stützplatte 6 sind quer zu einer Längsachse 9 des Reaktorbehälters 2 im Reaktorbehälter 2 angeordnet. Die erste Stützplatte 5 ist zur zweiten Stützplatte 6 entlang der Längsachse 9 des Reaktorbehälters 2 versetzt.

Jedes der Rohre 4 der ersten Rohrgruppen 7 ist durch eine jeweilige Rohröffnung 10.1 der ersten Stützplatte 5 geführt, und die erste Stützplatte 5 weist mehrere Aussparungen 11.1 zum Fluidaustausch auf. Jede der zweiten Rohrgruppen 8 ist durch eine jeweilige der Aussparungen 11.1 in der ersten Stützplatte 5 geführt.

Jedes der Rohre 4 der zweiten Rohrgruppen 8 ist durch eine jeweilige Rohröffnung 10.2 der zweiten Stützplatte 6 geführt, und die zweite Stützplatte 6 weist mehrere Aussparungen 11.2 zum Fluidaustausch auf. Jede der ersten Rohrgruppen 7 ist durch eine jeweilige der Aussparungen 11.2 in der zweiten Stützplatte 6 geführt.

Die erste Stützplatte 5 stützt die Rohre 4 der ersten Rohrgruppen 7 in den Rohröffnungen 10.1 der ersten Stützplatte 5 quer zur Längsrichtung 9 der Rohre 4 und die zweite Stützplatte 6 stützt die Rohre 4 der zweiten Rohrgruppen 8 in den Rohröffnungen 10.2 der zweiten Stützplatte 8 quer zur Längsrichtung 9 der Rohre 4.

Die Rohre 4 sind an einem ersten Ende 18 und an einem zweiten Ende 19 mit einer jeweiligen Rohrendplatte verbunden. Der Reaktorbehälter 2 und die Rohre 4 des Rohrbündels 3 sind aufrecht ausgerichtet. Das erste Ende 18 ist oben und das zweite Ende 19 ist unten im Reaktorbehälter 2 angeordnet.

Das Synthesegas wird in einem Verteiler 20 auf die Rohre 4 des Rohrbündels 3 verteilt.

In den Rohren 4 befindet sich ein Katalysator 30. Das Synthesegas enthält Reaktionsedukte. Die Reaktionsedukte für die Methanolsynthese werden durch die Rohre 4 des Rohrbündels 3 geleitet. Dabei wird das Synthesegas in den Rohren 4 teilweise zu Methanol umgesetzt. Ein Kühlmedium wird außerhalb der Rohre 4 des Rohrbündels 3 durch den Reaktorbehälter 2 geleitet. Die aus der exothermen Reaktion im Rohr 4 freiwerdende Wärme wird über die Rohrwand auf das in einem Mantelraum 24 befindliche Kühlmedium übertragen. Während der Katalysator 30 im Rohr 4 durch diesen Prozess gekühlt wird, wird dem Kühlmedium Energie zugeführt. Als Kühlmedium dient Wasser, welches dem Mantelraum 24 am zweiten Ende 19 zugeführt wird. Entstehende Dampfblasen und das siedende zweiphasige Wassergemisch strömen aufgrund von Dichteunterschieden vertikal nach oben. Dabei strömt der Wasserdampf durch die Aussparungen 11.1 der ersten Stützplatte 5 und durch die Aussparungen 11.2 der zweiten Stützplatte 6 mit geringen Druckverlusten. Der Wasserdampf wird oben gesammelt und einem Wasser-Kondensator 31 zugeführt. In dem Reservoir des Kondensators 31 befinden sich gesättigter Dampf 22 und Wasser 23 am bzw. leicht unter dem Siedepunkt. Das Wasser 23 wird der ersten Vorrichtung 1.1 am zweiten Ende 19 zugeführt und auf den Mantelraum 24 des Reaktorbehälters 2 verteilt. Die Kühlung funktioniert damit nach dem Thermosiphoneffekt.

Produktgas und das teilweise nicht reagierte Synthesegas werden aus dem Rohrbündel 3 in einem Sammler 21 gesammelt. Das Gasgemisch strömt anschließend zu der zweiten Vorrichtung 1.2.

In der zweiten Vorrichtung 1.2, welche der ersten Vorrichtung 1.1 nachgeschaltet ist, wird Synthesegas aus einem Synthesegaseinlass 25 vorgeheizt.

Die zweite Vorrichtung 1.2 ist teilweise analog zur ersten Vorrichtung 1.1 aufgebaut.

Im Unterschied zur ersten Vorrichtung 1.1, befindet sich in der zweiten Vorrichtung 1.2 zwischen den Rohren 4 der zweiten Vorrichtung 1.2 im Mantelraum 24 ein als Katalysatorschüttung vorliegender Katalysator 30.

Das Synthesegas, welches die Reaktionsedukte umfasst, wird für die Methanolsynthese außerhalb der Rohre 4 des Rohrbündels 3 durch den Reaktorbehälter 2 durch den Mantelraum 24 geleitet und ein Kühlmedium wird durch die Rohre 4 des Rohrbündels 3 geleitet. Dabei strömt das Synthesegas im Mantelraum 24 durch die Aussparungen 11.1 der ersten Stützplatte 5 und durch die Aussparungen 11.2 der zweiten Stützplatte 6 mit geringen Druckverlusten nach unten zu einem Produktgasauslass 28.

Die aus der exothermen Reaktion im Mantelraum 24 freiwerdende Wärme wird über die Rohrwand auf das im Rohr 4 befindliche Kühlmedium übertragen. Während der Katalysator 30 im Mantelraum 24 durch diesen Prozess gekühlt wird, wird dem Kühlmedium Energie zugeführt. Als Kühlmedium dient Synthesegas, welches den Rohren 4 über den Synthesegaseinlass 25 zugeführt wird. Durch die zugeführte Energie erwärmt sich das Synthesegas aus dem Synthesegaseinlass 25 und wird als vorgeheiztes Synthesegas 26 zum Gaseinlass 27 der ersten Vorrichtung 1.1 geführt.

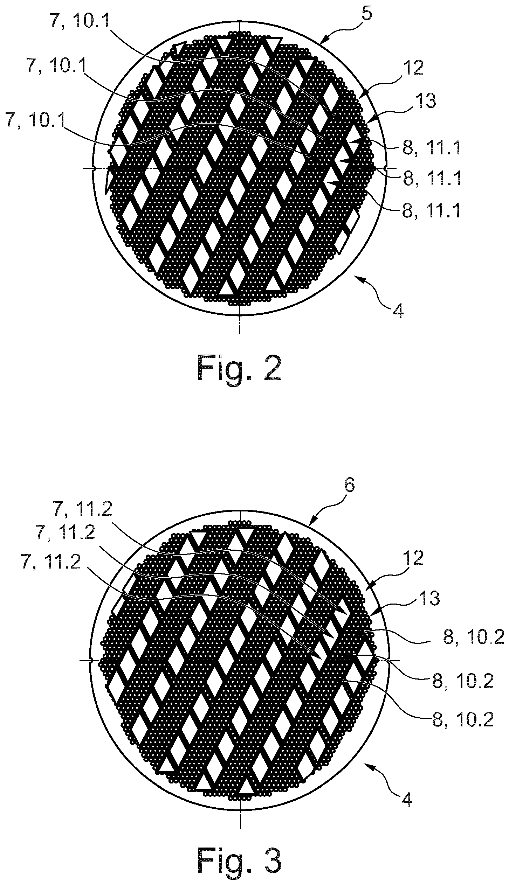

Fig. 2 zeigt eine schematische Draufsicht auf eine erste Ausgestaltung einer ersten Stützplatte 5, wie sie bei der Anordnung 29 aus Fig. 1 verwendet werden kann. Die Aussparungen 11.1 in der ersten Stützplatte 5 sind rautenförmig. Jeweils mehrere der ersten Rohrgruppen 7 sind in einer ersten Reihe 12 nebeneinander angeordnet. Jeweils mehrere der zweiten Rohrgruppen 8 sind in einer zweiten benachbarten Reihe 13 nebeneinander angeordnet. Erste Reihen 12 und zweite Reihen 13 sind alternierend angeordnet. Die Rohre 4 der zweiten Rohrgruppen 8 sind durch die Aussparungen 11.1 der ersten Stützplatte 5 geführt. Die Rohre 4 der ersten Rohrgruppen 7 sind durch die jeweiligen Rohröffnungen 10.1 der ersten Stützplatte 5 geführt. Ein Fluidaustausch wird durch die Aussparungen 11.1 im verbleibenden freien Strömungsquerschnitt zwischen den Rohren 4 der zweiten Rohrgruppen 8 ermöglicht.

Fig. 3 zeigt eine schematische Draufsicht auf eine erste Ausgestaltung einer ersten Stützplatte 6, wie sie bei der Anordnung 29 aus Fig. 1 verwendet werden kann. Die Aussparungen 11.2 in der ersten Stützplatte 6 sind rautenförmig. Jeweils mehrere der zweiten Rohrgruppen 8 sind in einer zweiten Reihe 13 nebeneinander angeordnet. Jeweils mehrere der ersten Rohrgruppen 7 sind in einer ersten benachbarten Reihe 12 nebeneinander angeordnet. Erste Reihen 12 und zweite Reihen 13 sind alternierend angeordnet. Die Rohre 4 der ersten Rohrgruppen 7 sind durch die Aussparungen 11.2 der ersten Stützplatte 6 geführt. Die Rohre 4 der zweiten Rohrgruppen 8 sind durch die jeweiligen Rohröffnungen 10.2 der zweiten Stützplatte geführt. Ein Fluidaustausch wird durch die Aussparungen 11.2 im verbleibenden freien Strömungsquerschnitt zwischen den Rohren 4 der ersten Rohrgruppen 7 ermöglicht.

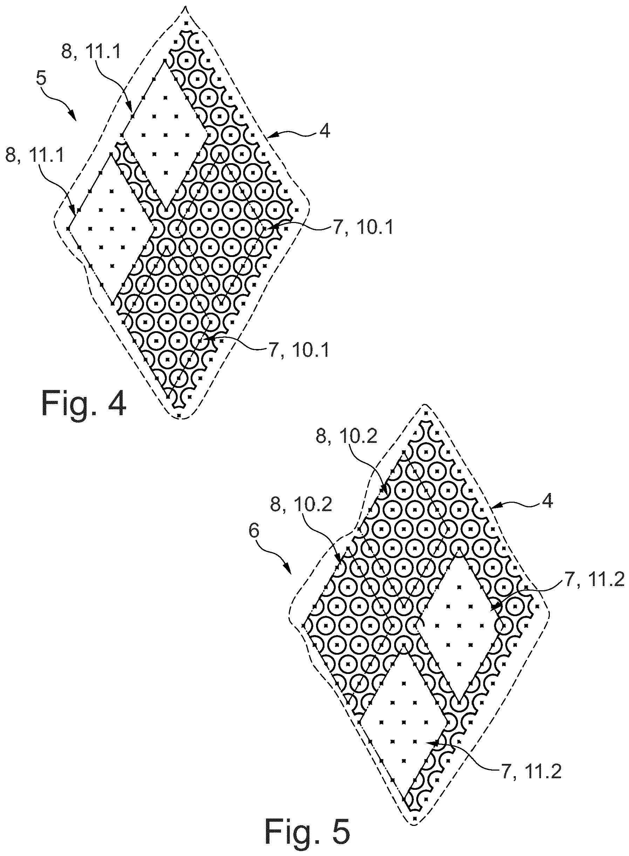

Fig. 4 zeigt eine Detailansicht einer ersten Stützplatte 5 und Fig. 5 zeigt einen Detailansicht einer zweiten Stützplatte 6 in einer ersten Ausgestaltung aus Fig. 2 und Fig. 3. Die Aussparungen 11.1 in der ersten Stützplatte 5 und die Aussparungen 11.2 in der zweiten Stützplatte 6 sind rautenförmig. Jeweils mehrere der ersten Rohrgruppen 7 sind nebeneinander angeordnet. Jeweils mehrere der zweiten Rohrgruppen 8 sind nebeneinander angeordnet. Die Rohre 4 der zweiten Rohrgruppen 8 sind durch die Aussparungen 11.1 der ersten Stützplatte 5 geführt. Die Rohre 4 der ersten Rohrgruppen 7 sind durch die jeweiligen Rohröffnungen 10.1 der ersten Stützplatte 5 geführt.

Die Rohre 4 der ersten Rohrgruppen 7 sind durch die Aussparungen 11.2 der zweiten Stützplatte 6 geführt. Die Rohre 4 der zweiten Rohrgruppen 8 sind durch die jeweiligen Rohröffnungen 10.2 der zweiten Stützplatte 6 geführt.

In Fig. 4 wird ein Fluidaustausch durch die Aussparungen 11.1 im verbleibenden freien Strömungsquerschnitt zwischen den Rohren 4 der zweiten Rohrgruppen 8 ermöglicht. In Fig. 5 wird ein Fluidaustausch durch die Aussparungen 11.2 im verbleibenden freien Strömungsquerschnitt zwischen den Rohren 4 der ersten Rohrgruppen 7 ermöglicht. In dieser Ausgestaltung umfassen die rautenförmigen Aussparungen 11.1 jeweils 5 mal 5 Rohre der zweiten Rohrgruppen 8 und die rautenförmigen Aussparungen 11.2 umfassen jeweils 5 mal 5 Rohre der ersten Rohrgruppen 7.

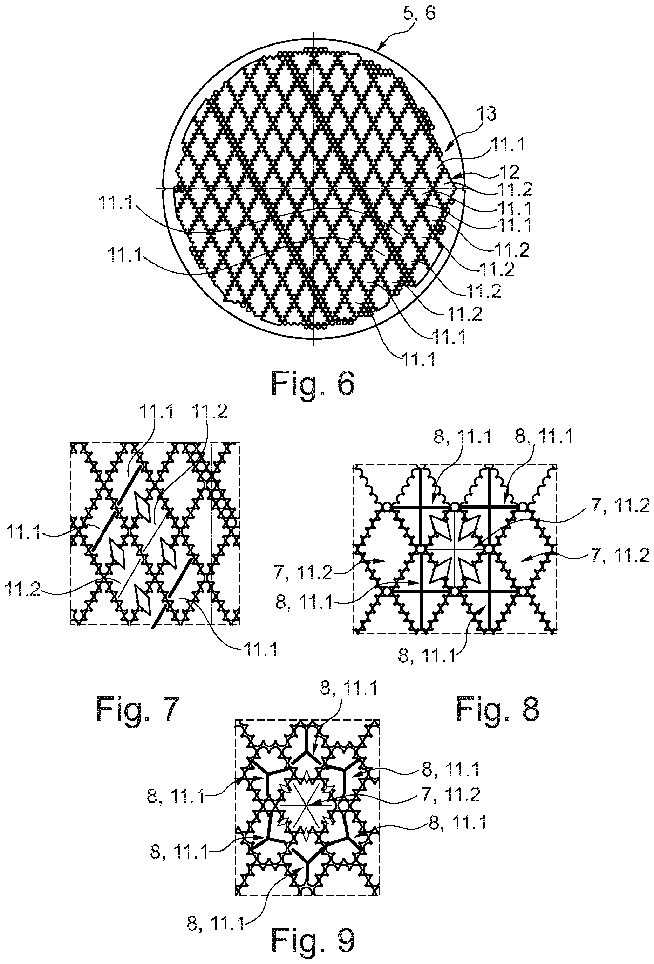

Fig. 6 zeigt eine schematische Draufsicht auf eine erste Stützplatte 5 mit Aussparungen 11.1 und auf eine zweite Stützplatte 6 mit Aussparungen 11.2 in einer überlagerten Betrachtungsweise in einer ersten Ausgestaltung aus Fig. 2 bis Fig. 5, wie sie bei der Anordnung 29 aus Fig. 1 verwendet werden kann.

In einer überlagerten Betrachtungsweise werden nur die Aussparungen 11.1, 11.2 der ersten und der zweiten Stützplatte 5, 6 berücksichtigt. Die erste Stützplatte 5 und die zweite Stützplatte 6 werden zu Darstellungszwecken in einer Ebene überlagert dargestellt. Dementsprechend können jeweils mehrere der Aussparungen 11.2 der zweiten Stützplatte 6 in einer ersten Reihe 12 nebeneinander angeordnet sein, wobei jeweils mehrere der Aussparungen 11.1 der ersten Stützplatte 5 in einer zweiten benachbarten Reihe 13 nebeneinander angeordnet sind, wobei erste und zweite Reihen 12, 13 alternierend angeordnet sind. Dies kann auch als ein Muster bezeichnet werden.

Fig. 7 zeigt eine Detailansicht der ersten Ausgestaltung der Aussparungen 11.1 der ersten Stützplatte 5 und der Aussparungen 11.2 der zweiten Stützplatte 6 in einer überlagerten Betrachtungsweise aus Fig. 6. Für den Fluidaustausch strömt ein Fluid durch die Aussparungen 11.1 der ersten Stützplatte 5 zu den nächstliegenden Aussparungen 11.2 der zweiten Stützplatte 6. Alternativ strömt ein Fluid zum Fluidaustausch durch die Aussparungen 11.2 der zweiten Stützplatte 6 zu den nächstliegenden Aussparungen 11.1 der ersten Stützplatte 5.

Fig. 8 zeigt eine Detailansicht einer zweiten Ausgestaltung der Aussparungen 11.1 der ersten Stützplatte 5 und der Aussparungen 11.2 der zweiten Stützplatte 6 in einer überlagerten Betrachtungsweise, wie sie bei der Anordnung 29 aus Fig. 1 verwendet werden kann. In einer zweiten Ausgestaltung der Aussparungen 11.1, 11.2 weisen die Aussparungen 11.1 in der ersten Stützplatte 5 jeweils eine sechseckige Form auf und/oder die Aussparung 11.2 in der zweiten Stützplatte 6 weisen jeweils eine sechseckige Form auf.

In dieser zweiten Ausgestaltung umfassen die sechseckigen Aussparungen 11.1 jeweils 34 Rohre der zweiten Rohrgruppen 8 und die sechseckigen Aussparungen 11.2 umfassen jeweils 34 Rohre der ersten Rohrgruppen 7. Die jeweilige sechseckige Form ist länglich ausgestaltet, mit 2 kurzen gegenüberliegenden Seiten und 4 langen Seiten.

Die ersten Rohrgruppen 7 und zweiten Rohrgruppen 8 sind in einem Schachbrettmuster alternierend angeordnet. In diesem Muster umgeben vier zweite Rohrgruppen 8 eine ersten Rohrgruppen 7 an den langen Seiten des Sechsecks. Dementsprechend können bei einer überlagerten Betrachtungsweise jeweils mehrere der Aussparungen 11.2 der zweiten Stützplatte 6 zusammen mit mehreren Aussparungen 11.1 der ersten Stützplatte 5 in einem Schachbrettmuster alternierend angeordnet sein.

Für den Fluidaustausch strömt ein Fluid durch die Aussparungen 11.1 der ersten Stützplatte 5 zu den nächstliegenden Aussparungen 11.2 der zweiten Stützplatte 6. Alternativ strömt ein Fluid zum Fluidaustausch durch die Aussparungen 11.2 der zweiten Stützplatte 6 zu den nächstliegenden Aussparungen 11.1 der ersten Stützplatte 5.

Fig. 9 zeigt eine Detailansicht einer dritten Ausgestaltung der Aussparungen 11.1 der ersten Stützplatte 5 und der Aussparungen 11.2 der zweiten Stützplatte 6 in einer überlagerten Betrachtungsweise, wie sie bei der Anordnung 29 aus Fig. 1 verwendet werden kann. Jeweils eine erste Rohrgruppe 7 ist von sechs zweiten Rohrgruppen 8 umgeben. Dementsprechend sind bei einer überlagerten Betrachtungsweise sechs Aussparung 11.1 der ersten Stützplatte 5 zur Durchführung der sechs zweiten Rohrgruppen 8 um die eine Aussparung 11.2 der zweiten Stützplatte 6 zur Durchführung der einen ersten Rohrgruppe 7 herum angeordnet.

Für den Fluidaustausch strömt ein Fluid durch die Aussparungen 11.1 der ersten Stützplatte 5 zu den nächstliegenden Aussparungen 11.2 der zweiten Stützplatte 6. Alternativ strömt ein Fluid zum Fluidaustausch durch die Aussparungen 11.2 der zweiten Stützplatte 6 zu den nächstliegenden Aussparungen 11.1 der ersten Stützplatte 5.

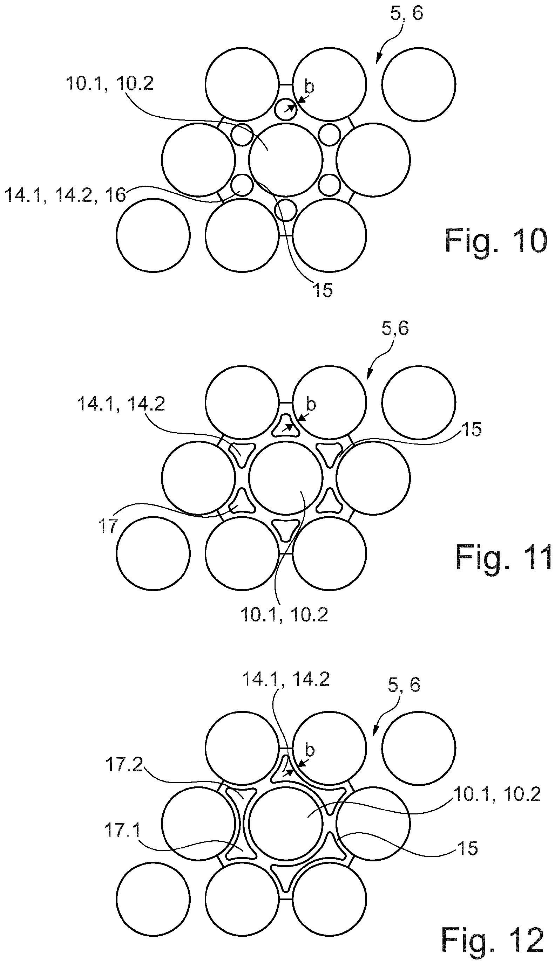

Fig. 10 zeigt eine Detailansicht einer vierten Ausgestaltung einer ersten Stützplatte 5 und einer zweiten Stützplatte 6, wie sie in den Vorrichtungen 1.1 1.2 der Fig. 1 eingesetzt werden kann. Die Stützplatten 5,6 bilden für zumindest einige Ausschnitte 14.1, 14.2 zwischen dem Ausschnitt 14.1, 14.2 und den dazu nächstliegenden der Rohröffnungen 10.1, 10.2 einen jeweiligen Steg 15 aus. Die Ausschnitte 14.1 sind der ersten Stützplatte 5 zugeordnet. Die Ausschnitte 14.2 sind der zweiten Stützplatte 6 zugeordnet. Die Stege 15 haben jeweils eine minimale Breite b, welche bei zumindest einigen der Stege 15 gleich groß ist. Die Ausschnitte 14.1, 14.2 liegen jeweils zentral zwischen drei paarweise zueinander benachbarten der Rohröffnungen 10.1, 10.2. Die Ausschnitte 14.1, 14.2 sind als Durchgangsbohrungen 16 gefertigt.

Fig. 11 zeigt eine Detailansicht einer dritten Ausgestaltung einer ersten Stützplatte 5 und einer zweiten Stützplatte 6, wie sie bei der Anordnung 29 aus Fig. 1 verwendet werden kann. Die erste Stützplatte 5 und die zweite Stützplatte 6 bilden für zumindest einige Ausschnitte 14.1, 14.2 zwischen dem Ausschnitt 14.1, 14.2 und den dazu nächstliegenden der Rohröffnungen 10.1, 10.2 einen jeweiligen Steg 15 aus. Die Ausschnitte 14.1 sind der ersten Stützplatte 5 zugeordnet. Die Ausschnitte 14.2 sind der zweiten Stützplatte 6 zugeordnet. Die Stege 15 haben jeweils eine minimale Breite b, welche bei allen der Stege 15 gleich groß ist. Die Ausschnitte 14.1, 14.2 liegen jeweils zentral zwischen drei paarweise zueinander benachbarten der Rohröffnungen 10.1, 10.2. Die erste Stützplatte 5 und die zweite Stützplatte 6weisen Zwischenbereiche 17 auf, welche jeweils zwischen drei paarweise zueinander benachbarten der Rohröffnungen 10.1, 10.2 ausgebildet sind. Die Ausschnitte 14.1, 14.2 der ersten Stützplatte 5 und der zweiten Stützplatte 6 sind auf einen jeweiligen der Zwischenbereiche 17 beschränkt. Die Zwischenbereiche 17 sind sternförm ig.

Fig. 12 zeigt eine Detailansicht einer vierten Ausgestaltung einer ersten Stützplatte 5 und einer zweiten Stützplatte 6, wie sie bei der Anordnung 29 aus Fig. 1 verwendet werden kann. Die erste Stützplatte 5und die zweite Stützplatte 6 bilden für zumindest einige Ausschnitte 14.1, 14.2 zwischen dem Ausschnitt 14.1, 14.2 und den dazu nächstliegenden der Rohröffnungen 10.1, 10.2 einen jeweiligen Steg 15 aus. Die Ausschnitte 14.1 sind der ersten Stützplatte 5 zugeordnet. Die Ausschnitte 14.2 sind der zweiten Stützplatte 6 zugeordnet. Die Stege 15 haben jeweils eine minimale Breite b, welche bei allen der Stege 15 gleich groß ist.

Die erste Stützplatte 5 und die zweite Stützplatte 6 weisen Zwischenbereiche 17.1; 17.2 auf, welche jeweils zwischen drei paarweise zueinander benachbarten der Rohröffnungen 10.1, 10.2 ausgebildet sind, und wobei sich zumindest einige der Ausschnitte 14.1, 14.2 der ersten Stützplatte 5 und der zweiten Stützplatte 6 jeweils über mindestens zwei benachbarte der Zwischenbereiche 17.1;17.2 erstrecken. Die Zwischenbereiche 17.1,17.2 sind sternförm ig.

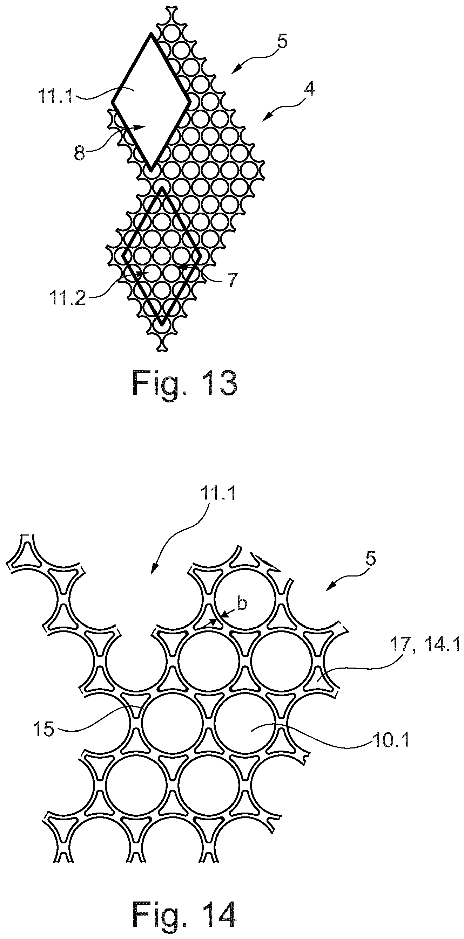

Fig. 13 und Fig. 14 zeigen eine Detailansicht einer siebten Ausgestaltung der Aussparungen und der Ausschnitte der ersten Stützplatte 5, wie sie bei der Anordnung 29 aus Fig. 1 verwendet werden kann.

Die erste Stützplatte 5 bildet für zumindest einige Ausschnitte 14.1 zwischen dem Ausschnitt 14.1 und den dazu nächstliegenden der Rohröffnungen 10.1 einen jeweiligen Steg 15 aus. Die Stege 15 haben jeweils eine minimale Breite b, welche bei allen der Stege 15 gleich groß ist. Die Ausschnitte 14.1 liegen jeweils zentral zwischen drei paarweise zueinander benachbarten der Rohröffnungen 10.1. Die erste Stützplatte 5 weist Zwischenbereiche 17 auf, welche jeweils zwischen drei paarweise zueinander benachbarten der Rohröffnungen 10.1 ausgebildet sind. Die Ausschnitte 14.1 der ersten Stützplatte 5 sind auf einen jeweiligen der Zwischenbereiche 17 beschränkt. Die Zwischenbereiche 17 sind sternförmig.

Die Aussparungen 11.1 in der ersten Stützplatte 5 sind rautenförmig. Die rautenförmigen Aussparungen 11.2 der zweiten Stützplatte 6 sind nur angedeutet. Jeweils mehrere der ersten Rohrgruppen 7 sind nebeneinander angeordnet. Jeweils mehrere der zweiten Rohrgruppen 8 sind nebeneinander angeordnet. Die Rohre 4 der zweiten Rohrgruppen 8 sind durch die Aussparungen 11.1 der ersten Stützplatte 5 geführt. Die Rohre 4 der ersten Rohrgruppen 7 sind durch die jeweiligen Rohröffnungen 10.1 der ersten Stützplatte 5 geführt.

Ein Fluidaustausch wird durch die Aussparungen 11.1 im verbleibenden freien Strömungsquerschnitt zwischen den Rohren 4 der zweiten Rohrgruppen 8 ermöglicht. In dieser Ausgestaltung umfassen die rautenförmigen Aussparungen 11.1 jeweils 5 mal 5 Rohre der zweiten Rohrgruppen 8.

### Bezugszeichenliste

- 1.1; 1.2: Vorrichtung
- 2: Reaktorbehälter
- 3: Rohrbündel
- 4: Rohr
- 5: erste Stützplatte
- 6: zweite Stützplatte
- 7: erste Rohrgruppen
- 8: zweite Rohrgruppen
- 9: Längsachse
- 10.1; 10.2: Rohröffnungen
- 11.1; 11.2: Aussparungen
- 12: erste Reihe
- 13: zweite Reihe
- 14.1; 14.2: Ausschnitt
- 15: Steg
- 16: Durchgangsbohrung
- 17; 17.1;17.2: Zwischenbereich
- 18: erstes Ende
- 19: zweites Ende
- 20: Verteiler
- 21: Sammler
- 22: gesättigter Dampf
- 23: Wasser leicht unter bzw. am Siedepunkt
- 24: Mantelraum
- 25: Synthesegaseinlass
- 26: vorgeheiztes Synthesegas
- 27: Gaseinlass
- 28: Produktgasauslass
- 29: Anordnung
- 30: Katalysator
- 31: Kondensator
- b: minimale Breite

## Patentansprüche

1. Vorrichtung (1.1;1.2) umfassend einen Reaktorbehälter (2), ein Rohrbündel (3) mit mehreren Rohren (4), eine erste Stützplatte (5) und eine zweite Stützplatte (6),
wobei das Rohrbündel (3) im Reaktorbehälter (2) angeordnet ist,
wobei das Rohrbündel (3) mehrere erste Rohrgruppen (7) und mehrere zweite Rohrgruppen (8) umfasst,
wobei die erste Stützplatte (5) und die zweite Stützplatte (6) quer zu einer Längsachse (9) des Reaktorbehälters (2) im Reaktorbehälter (2) angeordnet sind,
wobei die erste Stützplatte (5) zur zweiten Stützplatte (6) entlang der Längsachse (9) des Reaktorbehälters (2) versetzt ist,
wobei jedes der Rohre (4) der ersten Rohrgruppen (7) durch eine jeweilige Rohröffnung (10.1) der ersten Stützplatte (5) geführt ist, und wobei die erste Stützplatte (5) mehrere Aussparungen (11.1) zum Fluidaustausch aufweist,
wobei jede der zweiten Rohrgruppen (8) durch eine jeweilige der Aussparungen (11.1) in der ersten Stützplatte (5) geführt ist,
wobei jedes der Rohre (4) der zweiten Rohrgruppen (8) durch eine jeweilige Rohröffnung (10.2) der zweiten Stützplatte (6) geführt ist, und wobei die zweite Stützplatte (6) mehrere Aussparungen (11.2) zum Fluidaustausch aufweist, wobei jede der ersten Rohrgruppen (7) durch eine jeweilige der Aussparungen (11.2) in der zweiten Stützplatte (6) geführt ist,
wobei die erste Stützplatte (5) die Rohre (4) der ersten Rohrgruppen (7) in den Rohröffnungen (10.1) der ersten Stützplatte (5) quer zur Längsrichtung der Rohre (4) stützt und die zweite Stützplatte (6) die Rohre (4) der zweiten Rohrgruppen (8) in den Rohröffnungen (10.2) der zweiten Stützplatte (6) quer zur Längsrichtung der Rohre (4) stützt.

2. Vorrichtung (1.1; 1.2) nach Anspruch 1, wobei die Aussparungen (11.1) in der ersten Stützplatte (5) rautenförmig sind und/oder die Aussparungen (11.2) in der zweiten Stützplatte (6) rautenförmig sind.

3. Vorrichtung (1.1;1.2) nach einem der vorherigen Ansprüche,
wobei jeweils mehrere der ersten Rohrgruppen (7) in einer ersten Reihe (12) nebeneinander angeordnet sind, wobei jeweils mehrere der zweiten Rohrgruppen (8) in einer zweiten benachbarten Reihe (13) nebeneinander angeordnet sind, wobei erste und zweite Reihen (12; 13) alternierend angeordnet sind.

4. Vorrichtung (1.1;1.2) nach einem der vorherigen Ansprüche, wobei die erste Stützplatte (5) zusätzlich zu den Aussparungen (11.1) Ausschnitte (14.1) zum Fluidaustausch aufweist und/oder die zweite Stützplatte (6) zusätzlich zu den Aussparungen (11.2) Ausschnitte (14.2) zum Fluidaustausch aufweist.

5. Vorrichtung (1.1;1.2) nach Anspruch 4, wobei die erste Stützplatte (5) für zumindest einige der Ausschnitte (14.1) der ersten Stützplatte (5) jeweils zwischen dem Ausschnitt (14.1) und den dazu nächstliegenden der Rohröffnungen (10.1) der ersten Stützplatte (5) einen jeweiligen Steg (15) ausbildet, und wobei die Stege (15) jeweils eine minimale Breite (b) haben, welche bei zumindest einigen der Stege (15) gleich groß ist und/oder
die zweite Stützplatte (6) für zumindest einige der Ausschnitte (14.2) der zweiten Stützplatte (6) jeweils zwischen dem Ausschnitt (14.2) und den dazu nächstliegenden der Rohröffnungen (10.2) der zweiten Stützplatte (6) einen jeweiligen Steg (15) ausbildet, und wobei die Stege (15) jeweils eine minimale Breite (b) haben, welche bei zumindest einigen der Stege (15) gleich groß ist.

6. Vorrichtung (1.1;1.2) nach Anspruch 4 oder 5, wobei zumindest einige der Ausschnitte (14.1) der ersten Stützplatte (5) jeweils zentral zwischen drei paarweise zueinander benachbarten der Rohröffnungen (10.1) der ersten Stützplatte (5) liegen
und/oder
zumindest einige der Ausschnitte (14.2) der zweiten Stützplatte (6) jeweils zentral zwischen drei paarweise zueinander benachbarten der Rohröffnungen (10.2) der zweiten Stützplatte (6) liegen.

7. Vorrichtung (1.1;1.2) nach einem der Ansprüche 4 bis 6, wobei die erste Stützplatte (5) Zwischenbereiche (17.1; 17.2) aufweist, welche jeweils zwischen drei paarweise zueinander benachbarten der Rohröffnungen (10.1) ausgebildet sind, und wobei sich zumindest einige der Ausschnitte (14.1) der ersten Stützplatte (5) jeweils über zwei der Zwischenbereiche (17.1; 17.2) erstrecken,
und/oder
wobei die zweite Stützplatte (6) Zwischenbereiche (17.1; 17.2) aufweist, welche jeweils zwischen drei paarweise zueinander benachbarten der Rohröffnungen (10.2) ausgebildet sind, und wobei sich zumindest einige der Ausschnitte (14.2) der zweiten Stützplatte (6) jeweils über zwei der Zwischenbereiche (17.1; 17.2) erstrecken.

8. Anordnung (29) zur Methanolsynthese umfassend eine Vorrichtung (1.1; 1.2) nach einem der Ansprüche 1 bis 10.

9. Verwendung einer Vorrichtung (1.1; 1.2) nach einem der Ansprüche 1 bis 7 zur Methanolsynthese.

10. Verfahren zur Methanolsynthese mit einer Vorrichtung (1.1; 1.2) nach einem der Ansprüche 1 bis 7, wobei Reaktionsedukte für die Methanolsynthese durch die Rohre (4) des Rohrbündels (3) geleitet werden und ein Kühlmedium außerhalb der Rohre (4) des Rohrbündels (3) durch den Reaktorbehälter (2) geleitet wird,
oder
wobei Reaktionsedukte für die Methanolsynthese außerhalb der Rohre (4) des Rohrbündels (3) durch den Reaktorbehälter (2) geleitet werden und ein Kühlmedium durch die Rohre (4) des Rohrbündels (3) geleitet wird.
